# FASCICULE DE BREVET EUROPEEN

(11) **EP 2 582 283 B1**
(45) Date de publication et mention de la délivrance du brevet: **20.05.2015**
(21) Numéro de dépôt: 11728807.6
(22) Date de dépôt: 21.06.2011
(51) Int. Cl.: A61B 3/032, A61B 3/113, A61B 5/11, A61B 5/00, G02C 13/00

(54) **PROCEDE D'ESTIMATION D'UNE POSTURE DE REFERENCE**
VERFAHREN ZUR BEURTEILUNG EINER REFERENZHALTUNG
METHOD FOR ESTIMATING A REFERENCE POSTURE

(30) Priorité: 30.08.2010 FR 1056873; 21.06.2010 FR 1002603
(43) Date de publication de la demande: 24.04.2013
(73) Titulaire: INTERACTIF VISUEL SYSTEME (I V S), 92110 Clichy (FR)
(72) Inventeur: ENCAOUA, David, F-78420 Carrieres Sur Seine (FR); THOMET, Pascal, F-75006 Paris (FR)
(74) Mandataire: Regimbeau
(86) Numéro de dépôt international: PCT/EP2011/060304
(87) Numéro de publication internationale: WO 2011/161087

(56) Documents cités:
- EP-A1- 1 591 064
- EP-A1- 1 747 750
- FR-A1- 2 892 529

## Description

L'invention concerne l'analyse du comportement visuel d'un sujet, à des fins de personnalisation et d'optimisation des caractéristiques optiques de verres correcteurs ou de lentilles ophtalmiques qu'il doit porter, ainsi que leur montage sur la monture.

Plus précisément, l'invention concerne l'analyse de la posture générale de la tête du sujet et la prise de mesures effectuées par un opticien, qui procède à l'acquisition de données nécessaires afin de déterminer la configuration générale d'implantation des verres correcteurs en regard des yeux du sujet.

On connaît déjà de nombreux systèmes visant à optimiser la position des verres dans une monture par rapport à la position relative des pupilles des yeux du sujet et de la monture. A cet effet, des images fixes ou animées du visage portant la monture sont prises par une caméra, et la détection de la position des yeux est effectuée de même que la détection de l'emplacement de la monture.

En particulier, le document FR-2860887A au nom de la demanderesse divulgue un système dans lequel, à partir d'une série d'images animées du visage du sujet en cours de mouvement devant un caméra fixe, on détermine une image de référence dans laquelle le visage est le mieux axé sur la caméra, de manière à avoir la meilleure définition de la position relative des yeux et de la monture.

Parallèlement, des fabricants de verres ophtalmiques cherchent aujourd'hui à optimiser la conception de ces verres, notamment dans la technologie des verres dits progressifs, en examinant le comportement du sujet lorsque son regard se déplace. Par exemple, le document FR-2892529A au nom de la demanderesse divulgue un système comprenant :
- une caméra,
- un écran permettant de représenter les images prises par la caméra,
- un accessoire 20 apte à être porté de façon fixe par la tête du sujet et portant une pluralité de repères visuels 21,
- des moyens formant cible(s) visuelle(s) susceptibles de couvrir au moins deux positions déterminées par rapport à la caméra, et
- des moyens d'analyse d'image capables d'analyser la position des repères visuels dans les images prises par la caméra. Les moyens d'analyse d'image en déduisent alors la position et l'orientation dans l'espace de l'accessoire 20, et donc de la tête du sujet, lorsqu'il observe différentes régions des moyens formant cible(s) visuelle(s), pour en déduire notamment des informations sur l'importance relative du mouvement de la tête lors du déplacement de la vision d'une cible à l'autre, ainsi que l'importance relative du mouvement des yeux.

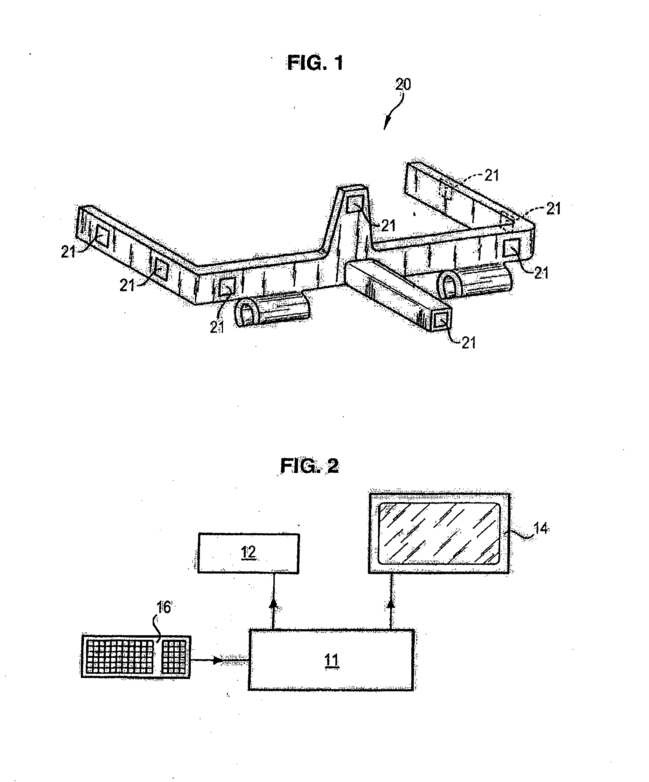

Typiquement, l'accessoire 20 peut être conforme à l'accessoire illustré en figure 1 et comprendre des moyens formant indicateurs géométriques 21 choisis spécifiquement pour mettre en valeur l'orientation de la monture. Il peut être constitué d'un support longiligne portant une série de repères visuels 21, placés le long des branches des montures et/ou sur le montant supérieur de face de la monture.

Pour que les mesures soient précises, il est nécessaire que la tête du sujet soit dans une posture de référence, et regarde dans une position définie, adaptée à la prise de mesures.

La posture de référence peut notamment être la posture de vision de loin, dans laquelle le porteur se tient dans une position naturelle et fixe un point à l'infini droit devant lui suivant un plan horizontal. En variante, la posture de référence peut correspondre à une posture de vision de près, telle que la position de lecture, c'est-à-dire la position dans laquelle le sujet fixe un point à environ quarante centimètres de ses yeux et abaisse sont regard de 30° par rapport au plan horizontal.

Dans la suite de cette description, la posture de référence correspondra à la posture en vision de loin. Ceci n'est cependant pas limitatif et n'est donné qu'à titre d'exemple.

Lorsque l'opticien mesure la position relative des pupilles par rapport à la monture, il est alors indispensable qu'il s'assure que le porteur soit dans une posture proche de cette posture de référence.

Or les systèmes connus à ce jour ne permettent pas de déterminer avec précision et de manière automatique si la posture du sujet est proche de la posture de référence. Cela est donc généralement fait manuellement, par l'opticien lui-même, à partir de la simple observation de la posture du sujet au moment de la prise des mesures. Pour cela, l'opticien peut notamment s'aider des systèmes décrits précédemment, qui donnent une valeur de l'inclinaison de l'accessoire fixé sur la monture.

La posture du sujet peut par exemple être décrite (de manière non limitative) à l'aide de deux angles pour une direction du regard connue. Le premier angle correspond au cap de la tête, c'est à dire un angle orienté qui reflète le fait que le sujet a tendance à avoir la tête plus ou moins tournée à gauche ou à droite lorsqu'il regarde un objet placé droit devant lui.

Le deuxième angle correspond à l'inclinaison de la tête, c'est à dire un angle orienté qui reflète le fait que le sujet a tendance à avoir la tête plus ou moins relevée ou abaissée lorsqu'il regarde un objet placé droit devant lui. Pour une monture donnée, ce deuxième angle peut être la mesure de l'angle pantoscopique, c'est à dire la mesure de l'inclinaison du plan moyen de la lentille correctrice par rapport à la verticale.

Afin de déterminer la distance entre les pupilles du sujet (un des paramètres pour la fabrication des dispositifs de correction), l'opticien utilise généralement un pupillomètre. Dans ce cas, le cap est arbitrairement supposé nul puisque le pupillomètre est en appui contre le front du sujet. Néanmoins, ce dispositif ne permet pas de tenir compte de l'angle pantoscopique qui doit être mesuré séparément, ni, le cas échéant, du fait que le sujet puisse présenter un port de tête latéralisé (tendance du sujet à regarder plutôt à droite ou à gauche dans sa position de référence).

Néanmoins, cette approche ne garantit pas que la position prise par le sujet corresponde bien à sa position de référence naturelle et n'est pas influencée par la présence notamment de l'opticien, d'un objet particulier dans la pièce ou de l'état psychologique instantané du sujet (effet du magasin qui peut intimider le sujet, contraintes liées aux demandes de l'opticien, etc.). Par exemple, si le porteur baisse trop la tête lors de la prise de mesures, on obtiendra des valeurs de mesures de hauteur trop fortes. Dans le cas de verres progressifs (pour lesquels la puissance de correction varie de haut en bas), il pourra alors avoir ses yeux en regard de la zone de correction de près, alors que l'objet qu'il regarde est au loin.

La posture de référence naturelle est cependant d'une grande importance car elle détermine la manière dont le porteur var projeter son regard sur les verres de lunettes dans sa position de confort maximum. Une mauvaise posture conduit donc à de mauvaises mesures de centrage des verres. Il est donc primordial de choisir une image posture correcte pour le calcul de la projection du regard sur les verres de lunette et donc d'évaluer la qualité de la posture du porteur pendant la mesure.

A titre d'illustration numérique, une erreur d'un degré sur l'orientation de la tête a pour conséquence une erreur de centrage d'environ 0.4 millimètres, alors que la précision recherchée doit être inférieure au demi millimètre.

Une approximation de l'inclinaison dans la posture de référence en vision de loin peut également être réalisée par référence au plan de Frankfort qui peut être défini par les tragions et le bas des orbites oculaires Une première approche pour mesurer le plan de Francfort est d'utiliser les deux centres de rotation de l'oeil pour déterminer un premier axe qui sera par définition parallèle au plan de Francfort et à une distance connue, d'environ 22 millimètres du plan de Frankfort. Ensuite on place manuellement, automatiquement ou d'une manière semi-assistée la position d'au moins un tragion sur au moins deux images du visage du sujet portant un accessoire 20 tel que celui défini plus haut. Les points du tragion étant des points de références (visibles) et le tragion étant immobiles dans le repère de l'accessoire 20, il peut être positionné en trois dimensions comme étant l'intersection des deux droites d'observation passant par les tragions et le centre optique de la caméra. Le plan de Francfort peut alors facilement être calculé comme étant le plan contenant le tragion et tangent au cylindre.

Ce plan est réputé horizontal lorsque le sujet est en position de vision de loin. Cette mesure est morphologique et indépendante de la posture du sujet pendant la mesure. Cependant cette approche ne garanti pas que la position naturelle en vision de loin coïncide parfaitement avec un plan de Frankfort horizontal.

Le document FR-2892529 décrit un procédé permettant de déterminer la répartition entre le mouvement de la tête et le mouvement des yeux du sujet lors d'un déplacement du regard entre deux cibles visuelles. Il s'agit donc dans ce document d'effectuer une mesure d'un paramètre donné du comportement visuel du porteur de lunettes ophtalmiques (mouvement relatif oeil/tête) et non de déterminer la posture de référence du porteur de lunettes ophtalmiques. Afin de mesurer ce paramètre, le document FR-2892529 propose de déterminer une position de référence du visage conformément à la description du document FR-2860887A, en identifiant une position de la tête du porteur où un accessoire, porté par le porteur, présente une position symétrique par rapport à un plan vertical. Il s'agit donc ici de déterminer une position de référence du visage pour mesurer le paramètre donné du comportement visuel du porteur. Une telle procédure ne permet cependant pas d'estimer la posture de référence naturelle du sujet.

Le document EP1591064 quant à lui décrit un procédé de détermination de l'angle entre la position de vision de loin et la position de vision de près d'un sujet. Il ne s'agit donc nullement d'estimer une posture de référence naturelle du sujet.

L'invention vise donc à proposer un procédé permettant de déterminer avec précision une position de référence naturelle du sujet, en tout ou partie automatique.

Pour cela, l'invention propose un procédé d'estimation d'une posture de référence d'un sujet conforme à la revendication 1.

L'enchaînement de ces étapes permet ainsi d'obtenir une posture de référence optimale, et ce malgré le fait que chaque diversion augmente les dispersions et donc la variabilité du port de tête. La mise en oeuvre de diversions répétées était donc quelque chose que l'homme du métier aurait cherché à éviter.

Certains aspects préférés mais non limitatifs du procédé selon l'invention sont les suivants :
- la posture de référence réelle est mesurée lors de l'arrivée de la tête sur la posture de référence cible ;
- au moins deux postures de référence réelles sont mesurées correspondant à deux postures de diversion opposées, et la posture de référence optimale est obtenue en effectuant une moyenne des postures de référence mesurées lors des ces deux postures de diversion opposées ;
- le procédé comprend en outre l'obtention d'un intervalle de confiance sur ladite posture de référence optimale ;
- la posture de référence cible correspond à une posture dans laquelle le sujet regarde naturellement une cible déterminée ;
- la posture de référence correspond à la posture de vision de loin ou de vision de près du sujet ;
- les données de posture comprennent une valeur d'angle pantoscopique et de cap de la tête du sujet ;
- les valeurs d'angle pantoscopique et de cap sont déterminées au moyen de probabilités conditionnelles dépendant des ensembles de données de posture de référence réelle précédents et/ou du type de posture de diversion précédente ;
- le procédé comprend en outre l'application d'un poids aux données en fonction de leur pertinence respective pour l'évaluation de la posture de référence optimale et de l'intervalle de confiance ;
- le procédé comprend en outre une étape de détermination du plan de Frankfort du sujet ;
- on intègre en outre des données du plan de Frankfort dans les données prises en compte pour la détermination de la posture de référence optimale et de l'intervalle de confiance ;
- le procédé comprend en outre l'extrapolation de données supplémentaires relatives à des postures de référence qui n'ont pas été prises par le sujet à partir des données enregistrées ;
- la mesure de la posture de référence réelle comprend la localisation de points singuliers sur au moins une image du sujet ;
- les points singuliers sont portés par un accessoire fixé sur une monture de lunettes portée par le sujet ;
- le procédé comprend en outre l'édition des données de posture utilisée pour le calcul des paramètres de fabrication du dispositif de correction, à partir :
- de l'intervalle de confiance et de la posture de référence optimale d'un ensemble de données d'une posture de référence réelle ou extrapolée à partir des ensembles de données réelles,
- de la présentation d'une image du sujet dans cette posture et/ou de l'impact du choix de cette posture sur les paramètres de fabrication du dispositif de correction.

Selon un deuxième aspect, l'invention propose un système d'estimation d'une posture de référence d'un sujet conforme à la revendication 13.

Certains aspects préférés mais non limitatifs du système sont les suivants :
- l'unité de traitement numérique permet en outre d'obtenir un intervalle de confiance sur ladite posture de référence optimale ; et
- le dispositif d'affichage comprend en outre des cibles visuelles.

Selon un dernier aspect, l'invention propose un produit programme d'ordinateur comprenant des instructions de code de programme pour l'exécution des étapes du procédé décrit ci-dessus, lorsque ledit programme est exécuté par un ordinateur.

D'autres caractéristiques, buts et avantages de la présente invention apparaîtront mieux à la lecture de la description détaillée qui va suivre et en regard des dessins annexés donnés à titre d'exemples illustratifs et sur lesquels :
La figure 1 représente une vue en perspective d'un exemple d'accessoire pouvant être utilisé dans la mise en oeuvre du système et du procédé selon l'invention,
La figure 2 représente un schéma bloc de différents composants du système de l'invention, et

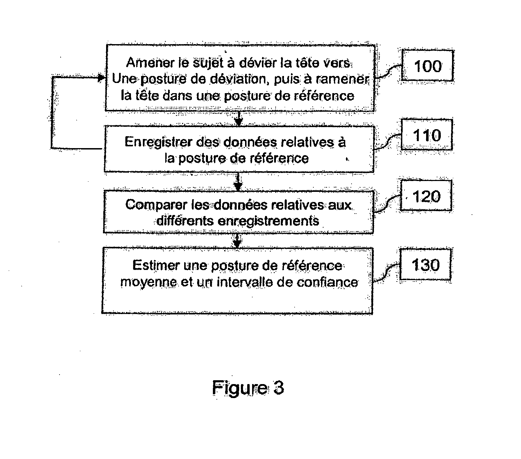

La figure 3 est une représentation graphique des différentes étapes selon une forme de réalisation de l'invention, et

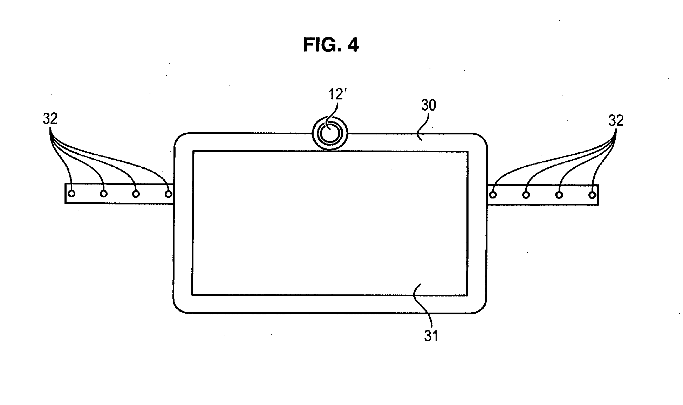

La figure 4 est une vue de face d'un exemple de système portable pouvant être utilisé dans la mise en oeuvre du système et du procédé selon l'invention.

Comme illustré sur la figure 2, un système conforme à l'invention comprend par exemple un bâti dans la région supérieure duquel sont logés un dispositif de capture d'images 12 tel qu'une caméra, placé à proximité d'un dispositif permettant d'obtenir une cible que le sujet va fixer dans la posture de référence, tels qu'un miroir sans tain placé devant la caméra. La caméra 12 est reliée à une unité centrale 11 pour l'acquisition des images vidéo. Un clavier 16, ainsi que tout autre dispositif d'entrée et de sortie tel que souris, un écran 14, etc. permet de commander le système.

Par exemple, il est possible d'intégrer l'invention dans le dispositif commercialisé sous le nom d'Activisu Expert 3 par la demanderesse.

Un tel dispositif comprend un miroir sans tain positionné verticalement. La caméra est placée derrière le miroir de sorte que la position de son axe optique principal par rapport au miroir soit connue. Typiquement, la caméra est disposée de sorte que son axe optique principal s'étende sensiblement perpendiculairement au miroir.

Par ailleurs, l'accessoire 20 a la forme d'un clip et est placé sur la monture.

Pour plus de détails sur le système, on pourra se référer par exemple aux demandes citées précédemment n°FR-2860887A ou FR-2892529A.

En variante, il n'est pas nécessaire de partir d'une image (ou toutes autre information utile aux mesures de centrage) pour chaque mesure de posture. Il est possible par exemple de mettre en oeuvre un système de suivi de la position de la tête en temps réel (ou calcul différé) associé à un appareil photo. L'étude de la posture sera alors réalisée avec un grand nombre de mesures de la posture alors qu'une seule (ou quelques) photo(s) sera(seront) prise(s) pour le centrage.

Des exemples de systèmes de suivi de la position de la tête peuvent être les suivants, de manière non limitative :
- Un accéléromètre placé de manière fixe par rapport à la tête. Une acquisition continue de l'accélération permet après un étalonnage approprié d'obtenir un suivi de la position de la tête.
- Un système par ultrason comme le Vision Print System commercialisé par la société Essilor, permettant de mesurer le coefficient oeil-tête pour la personnalisation du verre Ipseo.
- Des émetteurs infrarouges placés sur la tête du sujet, par exemple sur un accessoire 20 du type décrit précédemment ou sur tout objet fixe par rapport à la tête qui soit équivalent, associés à une caméra infrarouge. Le système est alors similaire au système d'Activisu Expert 3, mais met en oeuvre une caméra infrarouge au lieu d'une caméra sensible uniquement au spectre visible, et en utilisant des repères émettant dans le spectre infrarouge au lieu d'utiliser des repères visuels d'une couleur déterminée (vert sur le système Activisu Expert 3).
- Tout système de reconstruction tridimensionnelle du visage permettant d'obtenir un paramètre de posture suffisamment précis pour l'étude, ce paramètre pouvant être la rotation de la tête autour l'axe vertical (le cap) ou autour de l'axe horizontal orienté droite/gauche (lié à l'angle
pantoscopique). Cela peut être une reconstruction tridimensionnelle de points remarquables du visage par stéréoscopie (ou photogrammétrie) à l'aide de deux caméras (ou une reconstruction avec une seule caméra associée à un moyen de remise à l'échelle suffisamment précis pour l'étude (le moyen de remise à l'échelle pouvant être par exemple une distance connue entre deux des points remarquables identifiés sur une image).

Lors de la prise de mesure par l'un de ces dispositifs de suivi de la posture, on effectue en outre un lien entre ces mesures et une image du sujet afin de calculer la posture optimale

Afin de déterminer la posture naturelle du sujet, le procédé selon l'invention comprend notamment l'itération d'étapes réalisées au moyen de prises de vues (ou d'un dispositif équivalent de suivi de la position) et de calcul d'une posture optimale à partir de ces prises de vue.

Selon une forme de réalisation, le procédé comprend les étapes consistant à enregistrer 110 dans un premier temps une pluralité de postures du sujet grâce à la caméra 12, et, à chaque étape d'enregistrement, à faire effectuer au porteur au moins un mouvement déterminé avant de revenir à une posture de référence donnée 110.

Par exemple, les postures enregistrées peuvent correspondre à la posture à un instant donné du sujet lorsqu'il se regarde dans le miroir. Puis, par des moyens induisant un déplacement spontané tels que des instructions vocales, des messages affichés sur un écran, l'allumage d'une lumière dans un endroit donné de la pièce afin d'attirer le regard du sujet, ou plus généralement tout moyen amenant le patient à modifier sa posture, on induit 110 le déplacement du sujet ou du moins la rotation de sa tête avant de le ramener vers la position initiale de référence dans laquelle il se regarde dans le miroir.

Typiquement, il est possible de demander au sujet de tourner la tête à droite ou à gauche, de regarder au sol ou au plafond, de lire un document, de fermer les yeux, l'idée de base étant de le distraire temporairement afin de « réinitialiser » sa posture et de s'approcher de sa posture naturelle, malgré l'environnement et son état psychologique. On parvient ainsi à amener le sujet à placer son regard droit devant lui de manière généralement plus naturelle à chaque itération. L'amélioration n'est cependant pas garantie à chaque itération, dans la mesure où le sujet peut par exemple présenter une fatigue qui va l'amener à modifier sa position de référence. C'est pourquoi on privilégie l'utilisation d'une estimation statistique implémentée sur la base des différentes postures plutôt que la sélection de la dernière posture mesurée.

Dans le cas où la posture de référence correspond à la vision de près en position de lecture, le sujet fixe une cible et on effectue des mesures afin d'évaluer la distance entre la cible et la monture porté par le sujet, le cap, et l'angle formé par l'inclinaison du plan moyen des lentilles correctrices par rapport au plan contenant la direction du regard du sujet lorsqu'il fixe la cible (l'angle pantoscopique étant propre à la vision de loin).

Pour cela, la cible peut être sur une tablette 30, et la position de la monture est déterminée par l'intermédiaire de l'accessoire 20 fixé sur celle-ci.

Ici, la tablette 30 est un système portable doté d'un dispositif d'affichage 31, tel qu'un écran à cristaux liquides, et sur laquelle est monté un dispositif de prise d'image 12' tel qu'une caméra.

L'écran peut par exemple mesurer environ 40 cm de large pour 25cm de haut.

Avantageusement, cette tablette 30 permet ainsi de positionner le sujet par celle-ci. La personne peut donc se positionner face à tablette 30, et donc face à la caméra 12', la tablette 30 remplaçant avantageusement un miroir ou tout autre dispositif d'aide au positionnement.

Le sujet peut alors saisir la tablette 30 dans ses mains, comme un livre ou un journal, de telle sorte qu'il puisse fixer confortablement les éléments affichés par l'écran 31.

Il est par exemple possible d'afficher un ou plusieurs textes, des images, des publicités, fixes ou mobiles sur l'écran.

Typiquement, l'écran 31 peut afficher des informations sur les verres et lentilles existantes. Il peut également renvoyer au sujet l'image prise par la caméra, afin par exemple d'améliorer son centrage.

L'opérateur peut alors effectuer ses mesures, en demandant au sujet de visualiser les éléments affichés sur l'écran 31, puis un endroit de son choix dans la pièce, etc. de manière à déterminer, à l'aide de la caméra 12' montée sur l'écran 31 et/ou de la caméra 12, la posture de référence en position de lecture du sujet.

Selon une autre forme de réalisation, la tablette 30 comporte elle-même des moyens permettant de distraire le sujet afin de l'amener vers sa posture de référence naturelle.

Typiquement, la tablette 30 peut afficher un texte s'étendant sur toute la largeur de l'écran 31. De la sorte, lorsque le sujet lit le texte affiché, son regard parcourt l'écran et sa tête effectue un mouvement de rotation gauche/droite et haut/bas. Il est alors possible de déterminer la posture de référence du sujet, dans la mesure où le mouvement de la tête induit par la lecture du texte constitue une distraction suffisante pour réinitialiser la posture du sujet en position de lecture: en effet, lorsque celui-ci fixe ensuite un élément cible sur la tablette 30, telle qu'une image centrée ou une diode électroluminescente fixe par rapport à l'écran 31, sa position est plus naturelle qu'avant lecture.

De manière approximative, la posture de référence en position de lecture peut être déterminée comme étant la moyenne entre les postures extrêmes de lecture du texte sur l'écran 31.

Grâce à l'affichage du texte par l'écran 31, il est en outre possible d'effectuer une mesure d'un coefficient oeil-tête (c'est-à-dire un rapport moyen entre l'angle de rotation de l'oeil et la rotation de la tête du sujet), en déterminant, grâce à la caméra, l'intervalle angulaire de la tête du sujet : connaissant la distance entre la tête du sujet et la largeur de l'écran, on en déduit alors l'intervalle angulaire parcouru par les yeux du sujet au cours de la lecture, ainsi que le coefficient oeil-tête.

En variante, un texte est affiché à différents endroits de l'écran et/ou dans différentes tailles, de manière à amener le sujet à regarder différents endroits de l'écran avant de retourner sur l'élément cible. Une étude comportementale approfondie est en outre possible si la cible reste sensiblement ponctuelle (par exemple un texte très court) et se déplace en des positions variées de lecture (en haut, en bas, à droite ou à gauche), l'écran 31 étant piloté par l'unité de traitement 11, il est alors possible de savoir à tout moment comment évolue le port de tête en fonction des déplacements de la cible.

Selon une autre variante encore, outre les cibles visuelles affichées par l'écran 31, la tablette 30 peut comporter des cibles visuelles 32, telles que des diodes électroluminescentes (LED), fixes par rapport à l'écran. Un signal sonore peut accompagner l'allumage/l'extinction des cibles.

Par exemple, deux ensembles de LEDs peuvent être fixés sur des bras qui s'étendent radialement depuis des côtés latéraux de la tablette 30, de manière à créer trois ensembles de cibles visuelles 32 pour le sujet.

Ici, chaque bras comporte quatre LEDs, séparées les unes des autres d'une distance comprise entre approximativement 0.5 cm et 5 cm, de préférence environ 2 cm. Avantageusement, la mise en oeuvre d'une pluralité de LEDs adjacentes sur chaque bras permet d'adapter l'écart angulaire entre l'accessoire (et donc la tête du sujet) et la cible visuelle latérale (i.e. ici la LED allumée lors de la mesure) en fonction de la distance entre la tablette 30 et l'accessoire 20.

En variante (non illustré sur les figures), la tablette 30 ne comporte pas d'écran : elle comporte alors trois ensembles de LEDs, un central et deux latéraux.

Il suffit alors d'allumer successivement, de préférence de manière aléatoire, les cibles visuelles 32 afin de pouvoir effectuer les mesures du coefficient oeil-tête et du cap, ainsi que de déterminer la posture naturelle du sujet.

Au cours de ces étapes, on procède à l'acquisition et à l'analyse automatique des données enregistrées, et il n'est plus nécessaire de se référer à la seule expertise de l'opticien.

On procède alors au calcul de la position de référence « optimale » 130 à partir des observations et des enregistrements, position pouvant être différente des positions observées et correspondre à une position intermédiaire ou extrapolée.

Ce procédé est basé sur l'utilisation d'un modèle du comportement humain face au dispositif pour interpréter les mesures de postures.

Il peut notamment prendre la forme de probabilités conditionnelles, par exemple la probabilité d'avoir une posture de référence optimale *p* sachant que l'on dispose d'une séquence de postures *pi* mesurée après *i* itérations, et sachant les types de diversion utilisées (regarder en haut, à gauche, lire un document, etc.) préalablement à chaque posture *pi.* Par exemple des études ont montré que la probabilité que le cap de la tête soit bon est plus forte après avoir tournée la tête à droite puis à gauche qu'avant ces mouvements. Les mesures du cap obtenues après ces mouvements auront donc un poids plus élevé dans les calculs de cette valeur. Des considérations similaires peuvent-être avantageusement faite pour l'angle pantoscopique après avoir regardé le plafond

Le modèle du comportement humain face au dispositif pourra notamment utiliser l'éventail des outils mathématiques de statistiques de l'état de l'art et notamment :
- les probabilités conditionnelles, par exemple la probabilité d'avoir une posture *p* sachant que l'on dispose d'une séquence de postures *pi,* et dépendant des types de diversion utilisés (regarder en haut, à gauche, lire un document, etc.) préalablement à chaque posture *pi :* ces propriétés conditionnelles pourront être organisées dans une chaine de Markov.
- Des analyses avancées permettant de détecter si une valeur est « cohérentes » ou « aberrantes » et ainsi censurer les valeurs aberrantes
- Des réseaux bayesiens pourront par exemple être utilisés pour déterminer la séquence d'instruction de diversions à donner en fonction des postures précédemment observées et des instructions de diversion précédemment données.
- Les probabilités bayesiennes (ou tout autre outil statistique adapté) pourront permettre de modéliser par exemple la corrélation entre une posture ou un paramètre de posture externe, et les paramètres de postures de référence. Cette posture externe ou ce paramètre de posture externe pourra par exemple être : La posture en vision de lecture alors qu'on cherche à mesurer la posture en vision de loin. Le coefficient oeil-tête - mesuré par un système tel que Activisu Expert 3 développé par la demanderesse, ou encore le Vision Print system développé par Essilor - à savoir le pourcentage de rotation de la tête par rapport à la rotation de l'oeil lorsqu'on regarde un objet placé à 40° sur le côté de la tête.

Le résultat obtenu est sous la forme d'une valeur « optimale » et d'un intervalle de valeurs possibles.

Par valeur de posture, on comprendra ici notamment la valeur de l'angle pantoscopique et du cap pour une posture donnée.

Le modèle du comportement humain face au dispositif peut en outre être complété par un modèle « a priori », en particulier pour la mesure du cap, afin de réduire le nombre d'échantillons nécessaire pour détermination de la posture naturelle de référence du sujet. Un tel modèle « a priori » peut par exemple être utilisé pour améliorer le calcul du maximum de vraisemblance par inférences bayésiennes, en permettant notamment de compter sur une posture de référence plutôt qu'une autre, en particulier lorsque les postures prises par le sujet sont très instables, en se fondant sur des analyses statistiques.

Par exemple, on sait que le cap d'une personne est généralement plus proche de 0° que de 3°. Par conséquent, l'utilisation du modèle a priori implique de choisir une posture de référence proche de 0° plutôt que de 3° en cas d'instabilité des mesures.

Selon une forme de réalisation, l'utilisation d'un tel modèle a priori est progressive. De la sorte, plus les mesures effectuées sur le sujet sont stables, moins le modèle a priori est utilisé. Et *a contrario,* plus les mesures effectuées sont instables, plus le modèle a priori est privilégié, la moyenne des mesures n'étant pas suffisamment pertinente en raison de leur dispersion.

La détermination de la posture de référence naturelle « optimale » est alors plus fiable.

La mise en oeuvre du modèle a priori peut également être adaptée à la mesure de l'angle pantoscopique. Elle est cependant plus difficile que dans le cas de la mesure du cap, étant donné que l'angle pantoscopique peut varier entre 2 et 18°, au lieu de 0-3° pour le cap, et qu'il dépend entre autres de la monture et du port de tête du sujet. Typiquement, lorsque les mesures sont effectuées pour des verres progressifs, un modèle a priori peut privilégier des valeurs hautes pour l'angle pantoscopique afin de dégager la vision de loin du sujet, en prenant en compte de la posture après relèvement de la tête (lors du passage de la position de lecture à la position de loin).

L'opticien peut alors utiliser son expertise ou choisir la meilleure posture parmi l'espace de valeurs possibles proposées, et le système peut afficher en temps réel l'impact d'un choix de valeur de posture sur les résultats de mesures afin d'aider l'opticien dans sa décision.

En variante, l'affichage se fait de manière différée, par exemple à la fin de l'enregistrement de la série de postures *pi.*

Il est également possible d'afficher une image représentant la posture du porteur correspondant à une valeur de posture possible sélectionnée par l'opticien, afin de l'aider à vérifier que cette valeur correspond à la posture qu'il observe sur le porteur ou qu'il souhaite utiliser pour le centrage.

Cette image peut être l'image la plus représentative parmi celles enregistrées lors de la mesure (mesure de la posture et mesure de centrage). En variante, l'image affichée peut résulter de la production en image de synthèse de la posture, en effectuant une reconstruction tridimensionnelle à partir des images réelles et en interpolant les images enregistrées lors de l'itération.

Par exemple, il est possible de prendre la moyenne (qui pourra être pondérée par des critères de probabilités conditionnelles obtenus au moyen d'outils statistiques) comme valeur « optimale » et d'utiliser l'écart type pour calculer un intervalle de confiance comme intervalle de valeurs possibles.

De manière optionnelle, si la dispersion de l'angle pantoscopique (inclinaison haut/bas) est trop importante, on peut alors proposer à l'utilisateur de mesurer le plan de Frankfort, par exemple en déterminant la position de l'oeil et du tragion, et de l'utiliser pour améliorer les valeurs possibles et la valeur optimale.

Par défaut, on peut par exemple prendre la valeur du plan de Frankfort comme valeur optimale, ou l'utiliser pour détecter des valeurs de mesures aberrantes à censurer et réduire la dispersion. On peut en outre estimer la précision du plan de Frankfort et éventuellement en tenir compte dans les calculs statistiques, notamment avec un poids supérieur aux valeurs considérées comme moins pertinentes.

Par exemple, dans le cadre d'une mesure de la posture naturelle en vision de loin, l'invention peut comprendre les étapes suivantes.

L'opticien place le sujet devant le miroir.

De préférence, le sujet porte l'accessoire 20 formé de moyens formant indicateurs géométriques 21.

Des indications de « diversion » sont alors données au sujet (étape 100) : elles sont par exemple affichées sur l'écran et répétées par l'opticien, ou encore annoncées par haut-parleur, et l'opticien veille à ce qu'elles soient exécutées. Les instructions consisteront par exemple à demander de regarder le pont de la monture dans le miroir (ou tout autre cible pour la vision de loin) afin de déterminer une première posture de référence, puis à demander de suivre des indications de diversion afin d'amener le sujet dans une posture de diversion (tourner franchement la tête à droite ou à gauche, lever ou baisser franchement la tête, se tourner vers l'opticien (qui peut être placé derrière le sujet), fermer les yeux, se déplacer et se diriger vers un autre lieu avant de revenir face au miroir, etc.).

On demande alors au sujet de reprendre sa posture de référence, par exemple en fixant à nouveau le pont de la monture dans le miroir, et on enregistre la nouvelle posture de référence (étape 110).

Cette opération (diversion puis retour à la posture de référence et enregistrement de cette dernière posture de référence) est répétée au moins une fois, de préférence plusieurs fois.

En variante, la posture de référence enregistrée correspond à une posture de diversion du sujet, c'est-à-dire une posture dans laquelle celui-ci fixe une cible donnée (par exemple à droite ou en haut). L'opérateur demande alors au sujet de fixer une deuxième cible, dans la direction opposée (par exemple à gauche ou en bas, respectivement). La posture de référence du sujet (qui est prise en compte pour la détermination de la posture naturelle « optimale ») correspond alors à une moyenne (qui peut être pondérée) de ces deux postures de diversion.

Bien entendu, les postures peuvent être enregistrées en continu, sans que la posture de diversion pour laquelle la mesure est effectuée corresponde nécessairement à la position extrême de la diversion, grâce à la prise d'images en continu faite par la caméra. Ainsi, par exemple, il est possible de demander au sujet d'effectuer un mouvement continu de rotation droite/gauche de la tête et d'enregistrer deux postures de diversion, l'une à droite et l'autre à gauche, qui ne correspondent pas aux postures extrêmes du mouvement de rotation. De la sorte, on peut choisir par exemple des postures correspondant à la fixation de cibles légèrement décentrées à droite et à gauche (+/- 6° par exemple par rapport à la posture à 0°), et en faire la moyenne afin d'obtenir la posture de référence *pi* à prendre en compte pour les calculs de la valeur « optimale » de la posture de référence naturelle. Afin d'améliorer les mesures, il est même possible d'effectuer plusieurs mesures entre les deux points extrêmes des postures de diversion droite et gauche, puis de calculer une moyenne intégrée de ces mesures afin de déterminer la posture de référence *pi.*

Cette variante de réalisation présente l'avantage d'être moins brusque pour le sujet et plus fiable, puisque le sujet n'a plus besoin de repositionner sa tête au centre après chaque diversion. Par ailleurs, on privilégie des postures de diversions ayant une faible amplitude afin de rester dans l'intervalle de confort du sujet.

Avantageusement, les opérations de diversion diffèrent d'une itération à l'autre. Par exemple, il est possible de demander successivement au sujet de tourner la tête franchement à droite, puis à gauche, puis de regarder le plafond, le sol, de lire un document, le but étant de réinitialiser la posture de référence (ici, la posture correspondant à la vision de loin) afin de déterminer la posture naturelle du sujet.

Par ailleurs, le choix des diversions ainsi que l'ajustement des calculs peuvent dépendre du type de verre à réaliser pour la monture du sujet et de la stabilité de ses postures. Par exemple, les mesures effectuées pour la détermination de l'angle pantoscopique peuvent être privilégiées devant les mesures effectuées pour le cap dans le cas d'un verre progressif. En effet, les valeurs sont généralement davantage centrées pour le cap que pour l'angle pantoscopique, de sorte que leur dispersion est moindre.

Ce choix peut être fait préalablement, au début des mesures, ou au cours du procédé de mesure, en fonction des postures enregistrées. Ainsi, si on constate que la posture est instable lorsque le sujet relève ou baisse la tête, on privilégiera une diversion haut/bas supplémentaire afin de confirmer les mesures de l'angle pantoscopique, au détriment des autres.

On effectue alors une acquisition en temps réel en enregistrant les valeurs de posture *pi* après chaque diversion *i*, et en choisissant les indications de diversion en fonction des ports de tête déjà observés. Par exemple il est avantageux de demander au sujet de tourner plusieurs fois la tête à droite puis à gauche si son angle pantoscopique est très instable. En variante, il est également possible de demander au sujet de lever et de baisser la tête si la mesure de son cap montre qu'il est instable ; les inventeurs ont en effet constaté que, pour la mesure du cap, le sujet avait tendance à mieux replacer sa tête après avoir effectué un mouvement haut/bas ou bas/haut de la tête qu'après plusieurs mouvements droite/gauche. En effet, lors des mouvements de rotation droite/gauche de la tête, le sujet peut avoir tendance à résister ou au contraire amplifier le mouvement et s'arrêter au-delà de la posture naturelle lorsqu'il revient dans la position de référence, faussant ainsi les résultats, alors que lorsque le mouvement de rotation droite/gauche est suivi d'un mouvement haut/bas (ou bas/haut), le cap correspond sensiblement à celui de la position naturelle.

Les inventeurs ont également constaté que les diversions droite/gauche donnent une indication sur la stabilité et l'amplitude de la zone de confort du cap, tandis que les diversions haut/bas donnent une indication sur la stabilité et l'amplitude de la zone de confort de l'angle pantoscopique, et qu'une diversion haut/bas a tendance à recentrer le cap du sujet tandis qu'une diversion droite/gauche a tendance à recentrer son angle pantoscopique.

Connaissant la stabilité du cap et de l'angle pantoscopique du sujet, il est alors possible d'adapter le nombre de diversion ainsi que le type de diversions demandées à celui-ci afin de déterminer de manière optimale sa posture de référence.

On calcule alors la valeur optimale de la posture naturelle sur la base des différentes postures enregistrées *pi* et les valeurs possibles (étapes 120 et 130), par exemple les valeurs comprises dans un intervalle de confiance.

Durant le processus de mesures, le système pourra automatiquement détecter les instants où le porteur s'approche de la posture de référence (par opposition aux moments ou il en est éloigné, parce qu'il est en train de répondre à une instruction de « diversion »). Cette détection pourra notamment s'appuyer sur deux indices :
a. La posture est suffisamment proche de la posture de référence (limite sur l'angle de cap et angle pantoscopique, position dans l'espace délimité : distance au miroir, position haut bas droite et gauche)
b. La position est stabilisée : mouvements faibles pendant un lapse de temps déterminé.

Cette détection permettra d'agréger automatiquement les valeurs de postures à prendre en compte dans les calculs de statistiques et permettra d'avoir un processus automatisé.

Pour chaque posture il est possible de prendre la moyenne et l'écart type sur les images vidéo, d'extraire une tendance d'évolution sur la moyenne mobile, etc.

A partir de la valeur optimale de la posture naturelle, on détermine alors des résultats de mesures de centrage et de personnalisation (mesure de la distance entre les deux pupilles, la distance verre-oeil, etc.).

Selon une forme de réalisation, le système permet d'éditer la valeur de posture parmi les valeurs possibles.

Lors de ces éditions, l'opticien voit de manière interactive les nouvelles valeurs des mesures de centrage, la qualité des paramètres (cap et angle pantoscopique) choisis, et le cas échéant une image représentant la posture du porteur afin de vérifier que les paramètres correspondent à la posture qu'il observe sur le porteur ou qu'il souhaite utiliser pour le centrage.

Pour chaque posture il est possible de prendre la moyenne et l'écart type sur les images vidéo, extraire une tendance d'évolution sur la moyenne mobile etc.

On applique alors un poids aux valeurs en fonction d'un modèle de comportement donné. Par exemple un cap proche de zéro sera plus pertinent qu'une valeur élevée, , et un cap enregistré après un mouvement de rotation gauche/droite du visage suivi d'un mouvement haut/bas sera plus pertinent que celui mesuré directement suite à un mouvement de rotation gauche/droite, la diversion haut/bas ayant tendance à recentrer le cap du sujet.

De la même manière, l'angle pantoscopique sera plus naturel après que le porteur ait tournée la tête franchement vers le haut ou vers le bas, plus encore après qu'il ait effectué ces deux mouvements, etc. Par ailleurs, il est également possible d'appliquer un poids à chaque valeur en fonction du type de diversion effectué et du comportement (stabilité). Ainsi, pour l'évaluation du cap, les mesures effectuées suite à des diversions haut/bas peuvent avoir un poids plus important que les mesures effectuées directement après une diversion droite/gauche, puisqu'on a vu que ces premières donnent des résultats plus proches du cap naturel.

On affiche ensuite les résultats obtenus, par exemple sur l'écran du système.

Par ailleurs, il peut s'avérer avantageux d'offrir à l'opticien la possibilité d'éditer le port de tête de référence parmi un ensemble de valeurs acceptables et d'en apprécier l'impact sur les mesures de centrage. Afin d'évaluer qualitativement la pertinence du port de tête choisi, le système permet l'affichage d'une image représentative du port de tête de référence parmi les images enregistrées pendant la phase d'analyse, recalée sur la position des yeux (pour que le passage d'une image à l'autre soit continu) et interpolée pour simuler une position entre deux (ou plus) photos enregistrées. Des techniques telle que l'optical flow ou disparity map associées à du warping ou triangularisation et texture mapping peuvent être utilisées.

Les images de postures peuvent être extrapolées pour simuler une position qui n'a pas pu être enregistrée (sujet ayant un port de tête qui n'est pas du tout naturel devant le miroir). Cette extrapolation peut notamment être réalisée à partir du modèle a priori.

Elle peut par ailleurs être affichée en stéréoscopie tridimensionnelle pour mieux la percevoir.

## Revendications

1. Procédé d'estimation d'une posture de référence d'un sujet en vue de réaliser des mesures pour la détermination de paramètres pour la fabrication d'un dispositif de correction de vue, comprenant les étapes consistant, pour une pluralité d'arrivées de la tête sur une posture de référence cible à partir d'au moins une posture de diversion :
- lors de chaque arrivée sur la posture de référence cible, mesurer la posture de référence réelle, pour ainsi obtenir plusieurs ensembles de données de posture de référence réelle, et mémoriser lesdits ensembles de données,
- à l'aide d'une unité de traitement numérique, traiter lesdits ensembles de données de posture de référence réelle pour obtenir une posture de référence optimale.

2. Procédé selon la revendication 1, dans lequel la posture de référence réelle est mesurée lors de l'arrivée de la tête sur la posture de référence cible.

3. Procédé selon la revendication 1, dans lequel au moins deux postures de référence réelles sont mesurées correspondent à deux postures de diversion opposées, et la posture de référence optimale est obtenue en effectuant une moyenne des postures de référence mesurées lors des ces deux postures de diversion opposées.

4. Procédé selon l'une des revendications 1 et 2, comprenant en outre la détermination d'un intervalle de confiance sur ladite posture de référence optimale.

5. Procédé selon l'une des revendications 1 à 4, dans lequel la posture de référence cible correspond à une posture dans laquelle le sujet regarde naturellement une cible déterminée, ou à la posture de vision de loin ou de vision de près du sujet.

6. Procédé selon l'une des revendications 1 à 5, dans lequel les données de posture comprennent une valeur d'angle pantoscopique et de cap de la tête du sujet.

7. Procédé selon la revendication 6, dans lequel les valeurs d'angle pantoscopique et de cap sont déterminées au moyen de probabilités conditionnelles dépendant des ensembles de données de posture de référence réelle précédents et/ou du type de posture de diversion précédente.

8. Procédé selon l'une des revendications 1 à 7, comprenant en outre l'application d'un poids aux données en fonction de leur pertinence respective et du type de diversion associé pour l'évaluation de la posture de référence optimale.

9. Procédé selon l'une des 1 à 8 précédentes, comprenant en outre une étape de détermination du plan de Frankfort du sujet, et dans lequel on intègre en outre des données du plan de Frankfort dans les données prises en compte pour la détermination de la posture de référence optimale et de l'intervalle de confiance.

10. Procédé selon l'une des revendications 1 à 9, comprenant en outre la prise en compte progressive d'un modèle a priori.

11. Procédé selon l'une des revendications 1 à 10, comprenant en outre l'extrapolation de données supplémentaires relatives à des postures de référence qui n'ont pas été prises par le sujet à partir des données enregistrées.

12. Procédé selon l'une des revendications 1 à 11, comprenant en outre l'édition des données de posture utilisée pour le calcul des paramètres de fabrication du dispositif de correction, à partir
- de l'intervalle de confiance et de la posture de référence optimale d'un ensemble de données d'une posture de référence réelle ou extrapolée à partir des ensembles de données réelles,
- de la présentation d'une image du sujet dans cette posture et/ou de l'impact du choix de cette posture sur les paramètres de fabrication du dispositif de correction.

13. Système d'estimation d'une posture de référence d'un sujet adapté pour être mis en oeuvre dans un procédé selon l'une des revendications 1 à 12, comprenant :
- des moyens pour mesurer (12, 12'), lors de chaque arrivée sur la posture de référence cible, la posture de référence réelle, pour ainsi obtenir plusieurs ensembles de données de posture de référence réelle,
- des moyens pour mémoriser lesdits ensembles de données,
- une unité de traitement numérique (11) adaptée pour traiter lesdits ensembles de données de posture de référence réelle pour obtenir une posture de référence optimale,
le système étant **caractérisé en ce qu'**il comprend en outre un dispositif d'affichage (30) portable adapté pour être porté par le sujet, et **en ce que** les moyens de mesure (12, 12') comprennent un dispositif de capture d'image (12), fixe par rapport au dispositif d'affichage portable (30).

14. Système selon la revendication 13, **caractérisé en ce que** l'unité de traitement numérique est en outre adaptée pour obtenir un intervalle de confiance sur ladite posture de référence optimale.

15. Système selon la revendication 13, **caractérisé en ce que** le dispositif d'affichage (30) comprend en outre des cibles visuelles (32).

16. Produit programme d'ordinateur comprenant des instructions de code de programme pour l'exécution des étapes du procédé selon l'une des revendications 1 à 12, lorsque ledit programme est exécuté par un ordinateur.

## Patentansprüche

1. Verfahren zum Schätzen einer Referenzhaltung einer Person mit dem Ziel Messungen zum Bestimmen von Parametern zur Herstellung einer Vorrichtung zur Sehkorrektur auszuführen, das die Schritte umfasst, die für mehrere Vorgänge, in denen der Kopf ausgehend von wenigstens einer verdrehten Haltung eine Ziel-Referenzhaltung erreicht, Folgendes umfassen:
- bei jedem Erreichen der Ziel-Referenzhaltung, Messen der tatsächlichen Referenzhaltung, um dadurch mehrere Datensätze für die tatsächliche Referenzhaltung zu erhalten, und Speichern der Datensätze,
- mittels einer Einheit zur digitalen Verarbeitung, Verarbeiten der Datensätze für die tatsächliche Referenzhaltung, um eine optimale Referenzhaltung zu erhalten.

2. Verfahren nach Anspruch 1, bei dem die tatsächliche Referenzhaltung gemessen wird, wenn der Kopf die Ziel-Referenzhaltung erreicht.

3. Verfahren nach Anspruch 1, bei dem wenigstens zwei tatsächliche Referenzhaltungen gemessen werden, die zu zwei entgegengesetzten verdrehten Haltungen gehören, und die optimale Referenzhaltung erhalten wird, indem ein Mittelwert der Referenzhaltungen gebildet wird, die für diese beiden entgegengesetzten verdrehten Haltungen gemessen werden.

4. Verfahren nach einem der Ansprüche 1 und 2, das außerdem das Bestimmen eines Vertrauensintervalls für die optimale Referenzhaltung umfasst.

5. Verfahren nach einem der Ansprüche 1 bis 4, bei dem die Ziel-Referenzhaltung einer Haltung entspricht, bei der die Person auf natürliche Weise ein festgelegtes Ziel anschaut, oder der Haltung für die Fernsicht oder die Nahsicht der Person.

6. Verfahren nach einem der Ansprüche 1 bis 5, bei dem die Haltungsdaten einen Wert für den pantoskopischen Winkel und den Winkel für die Kopfausrichtung der Person umfassen.

7. Verfahren nach Anspruch 6, bei dem die Werte für den pantoskopischen und den Ausrichtungswinkel mittels bedingter Wahrscheinlichkeiten bestimmt werden, die von den vorhergehenden Datensätzen zur tatsächliche Referenzhaltung und/oder von der Art der vorhergehenden verdrehten Haltung abhängen.

8. Verfahren nach einem der Ansprüche 1 bis 7, das zum Ermitteln der optimalen Referenzhaltung außerdem die Anwendung eines Gewichts auf die Daten umfasst, und dies in Abhängigkeit von ihrer jeweiligen Zuverlässigkeit und der Art der zughörigen Verdrehung.

9. Verfahren nach einem der vorhergehenden Ansprüche 1 bis 8, das außerdem einen Schritt zum Bestimmen der Frankfurter Horizontalebene der Person umfasst und bei dem außerdem Daten zur Frankfurter Horizontalebene in die Daten integriert werden, die zum Bestimmen der optimalen Referenzhaltung und des Vertrauensintervalls berücksichtigt werden.

10. Verfahren nach einem der Ansprüche 1 bis 9, das außerdem die progressive Berücksichtigung eines Apriori-Modells umfasst.

11. Verfahren nach einem der Ansprüche 1 bis 10, das außerdem die Extrapolation zusätzlicher Daten umfasst, die Referenzhaltungen betreffen, die von der Person nicht erfasst wurden, und dies ausgehend von gespeicherten Daten.

12. Verfahren nach einem der Ansprüche 1 bis 11, das außerdem das Bearbeiten der Haltungsdaten umfasst, die zum Berechnen der Parameter zum Herstellen der Korrekturvorrichtung verwendet werden, und dies ausgehend von:
- dem Vertrauensintervall und der optimalen Referenzhaltung für einen Datensatz für eine tatsächliche oder ausgehend von den tatsächlichen Datensätzen extrapolierte Referenzhaltung,
- dem Darstellen eines Bildes der Person in dieser Haltung und/oder dem Einfluss der Auswahl dieser Haltung auf die Parameter zum Herstellen der Korrekturvorrichtung.

13. System zum Schätzen einer Referenzhaltung einer Person, das dafür eingerichtet ist, in einem Verfahren nach einem der Ansprüche 1 bis 12 eingesetzt zu werden, Folgendes umfassend:
- ein Mittel zum Messen (12, 12'), bei jedem Erreichen der Ziel-Referenzhaltung, der tatsächlichen Referenzhaltung, um dadurch mehrere Datensätze für die tatsächliche Referenzhaltung zu erhalten,
- ein Mittel zum Speichern der Datensätze,
- eine Einheit zur digitalen Verarbeitung (11), die dafür eingerichtet ist, die Datensätze für die tatsächliche Referenzhaltung zu verarbeiten, um eine optimale Referenzhaltung zu erhalten,
wobei das System **dadurch gekennzeichnet ist, dass** es außerdem eine tragbare Anzeigevorrichtung (30) umfasst, die dafür eingerichtet ist, von der Person gehalten zu werden, und dadurch, dass das Messmittel (12, 12') eine Vorrichtung zur Bilderfassung (12) umfasst, die relativ zur tragbaren Anzeigevorrichtung (30) feststehend ist.

14. System nach Anspruch 13, **dadurch gekennzeichnet, dass** die Einheit zur digitalen Verarbeitung außerdem dafür eingerichtet ist, ein Vertrauensintervall für die optimale Referenzhaltung zu erhalten.

15. System nach Anspruch 13, **dadurch gekennzeichnet, dass** die Anzeigevorrichtung (30) außerdem visuelle Ziele (32) umfasst.

16. Programmprodukt für einen Computer, das Programmcode-Anweisungen umfasst, um die Schritte des Verfahrens nach einem der Ansprüche 1 bis 12 auszuführen, wenn das Programm auf einem Computer ausgeführt wird.

## Claims

1. A process for estimation of a reference posture of a subject for the purposes of taking measurements for determination of parameters for the manufacture of a vision correction device, comprising the following steps, for a plurality of arrivals of the head on a target reference posture from at least one deviated posture:
- during each arrival on the target reference posture, measuring the real reference posture, to obtain several sets of data of real reference posture, and storing said sets of data,
- by means of a digital processing unit, processing said sets of data of real reference posture to obtain an optimal reference posture.

2. The process according to claim 1, wherein the real reference posture is measured during arrival of the head on the target reference posture.

3. The process according to claim 1, wherein at least two real reference postures are measured, said at least two real reference postures corresponding to two opposite deviated postures, and the optimal reference posture is obtained by taking an average of the reference postures measured during these two opposite deviated postures.

4. The process according to one of claims 1 and 2, further comprising the determination of a confidence interval on said optimal reference posture.

5. The process according to one of claims 1 to 4, wherein the target reference posture corresponds to a posture wherein the subject naturally looks at a determined target, or to the distant vision or near vision posture of the subject.

6. The process according to one of claims 1 to 5, wherein the posture data comprise a pantoscopic angle value and cap angle value of the head of the subject.

7. The process according to claim 6, wherein the pantoscopic angle value and cap angle value are determined by means of conditional probabilities depending on the previous sets of data of real reference posture and/or the previous type of deviated posture.

8. The process according to one of claims 1 to 7, further comprising the application of weight to the data as a function of their respective relevance and of the type of associated deviation for evaluation of the optimal reference posture.

9. The process according to one of the preceding claims 1 to 8, further comprising a step for determining the Frankfurt plane of the subject, and wherein data of the Frankfurt plane are further integrated into the data taken into account for determination of the optimal reference posture and the confidence interval.

10. The process according to one of claims 1 to 9, further comprising the progressive taking into account of an a priori model.

11. The process according to one of claims 1 to 10, further comprising the extrapolation of supplementary data relative to reference postures which have not been taken by the subject from recorded data.

12. The process according to one of claims 1 to 11, further comprising the edition of posture data used for calculating the production parameters of the correction device, from
- the confidence interval and the optimal reference posture of a set of data of a real reference posture or extrapolated from the sets of real data,
- presentation of an image of the subject in this posture and/or the impact of the choice of this posture on the production parameters of the correction device.

13. A system for estimating a reference posture of a subject adapted to be implemented in a process according to one of claims 1 to 12, comprising:
- means for measuring (12, 12'), during each arrival on the target reference posture, the real reference posture to obtain several sets of data of real reference posture,
- means for storing said sets of data,
- a digital processing unit (11) adapted to process said sets of data of real reference posture to obtain an optimal reference posture,
the system being **characterized in that** it further comprises a portable display device (30) adapted to be worn by the subject, and **in that** the measuring means (12, 12') comprise an image-capture device (12) fixed relative to the portable display device (30).

14. The system according to claim 13, **characterized in that** the digital processing unit is further adapted to produce a confidence interval on said optimal reference posture.

15. The system according to claim 13, **characterized in that** the display device (30) further comprises visual targets (32).

16. A computer program product comprising program code instructions for execution of the steps of the process according to one of claims 1 to 12, when said program is executed by a computer.
